# EUROPÄISCHE PATENTANMELDUNG

(11) **EP 1 900 384 A1**
(43) Veröffentlichungstag der Anmeldung: **19.03.2008**
(21) Anmeldenummer: 06120761.9
(22) Anmeldetag: 15.09.2006
(51) Int. Cl.: A61M 1/34, A61M 1/36, A61M 1/16

(54) **Therapieeinrichtung mit gedächtnisgestützter Regeleinrichtung**

(71) Anmelder: B. Braun Avitum AG, 49215 Glandorf (DE)
(72) Erfinder: Röher, Otfried, Professor Dr.-Ing., 01139 Dresden (DE); Korth, Steffen, Dipl.-Ing., 99334 Elleben (DE)
(74) Vertreter: Selting, Günther

(57) **Zusammenfassung**

Die Therapieeinrichtung führt eine Speicherung der Zeitverläufe des Blutdrucks und weiterer Einflussgrößen während vorangegangener Behandlungen durch. Auch der Zeitverlauf des Blutdrucks sowie der weiteren Einflussgrößen während der gegenwärtigen Behandlung wird erfasst. Es erfolgt ein analytischer Vergleich der Zeitverläufe des Blutdrucks und der weiteren Einflussgrößen während des gegenwärtigen Therapievorganges und während vorangegangener Therapievorgänge. In Abhängigkeit von dem Vergleich wird eine vorausschauende Blutdruckregelung zur Prävention von Blutdruckabfällen in einem nachfolgenden Zeitabschnitt durchgeführt.

## Beschreibung

Die Erfindung betrifft eine Therapieeinrichtung mit einem Blutdruckmessgerät und einer gedächtnisgestützten Regeleinrichtung zur Steuerung der Zeitpunkte der Blutdruckmessungen und zur Regelung des Blutdruckes des Patienten während eines Therapievorganges, insbesondere ein Therapiegerät zur extrakorporalen Blutreinigung.

Bei verschiedenen Therapien, wie beispielsweise bei der Hämodialyse, ist es erforderlich, den Blutdruck des Patienten laufend zu überwachen. Hierfür werden Geräte zur unblutigen (non-invasiven) Blutdruckmessung mit einer um den Arm des Patienten herumlegbaren Manschette benutzt, die aufgepumpt und dann unter Druckmessung langsam entlastet wird. Um eine quasikontinuierliche Blutdruckregelung zu erreichen, muss die Regeleinrichtung in Intervallen von wenigen Minuten Regelvorgänge durchführen. Die während einer mehrstündigen Behandlung hierfür erforderliche Anzahl von Blutdruckmessungen kann erheblich reduziert werden, wenn zu festgelegten Zeitpunkten die Blutdruckmessungen durch Blutdruckwerte substituiert werden, die aus früheren Behandlungen desselben Patienten gewonnen wurden.

In U.S. 6,578,241 B2 und EP 1 226 838 A2 sind entsprechende Therapieeinrichtungen mit intervallartiger Blutdruckmessung und mit reduzierter Anzahl der Messvorgänge beschrieben. Anhand der gleichen Länge aller Intervalle, beispielsweise 5 Minuten je Intervall, gewährleistet hierbei die Regeleinrichtung die Auswertung des Blutdruckverlaufes und der Blutdrucktrends in jedem Intervall nach einheitlichen Regeln, wie dies in EP 0 956 872 A2 beschrieben ist.

Da für morbide Ereignisse während der Hämodialyse, wie Hypotonie, Schwindelanfälle, Erbrechen u. a., multifaktorielle Ursachen zu berücksichtigen sind, werden in der klinischen Praxis mitunter Monitore für weitere Größen (beispielsweise EKG, relatives Blutvolumen, Hämatokrit, Blutsauerstoffgehalt) zur Erkennung von Komplikationen verwendet. Hieraus können sich in Abhängigkeit von den Messergebnissen zu beliebigen Zeitpunkten Anforderungen für zusätzliche Blutdruckmessungen ergeben. Klinische Erfahrungen mit Dialysepatienten zeigen, dass in Abhängigkeit vom Zustand des Patienten mitunter auch Blutdruckmessungen direkt vom medizinischen Personal angefordert werden. Eine entsprechende Therapieeinrichtung mit zeitflexibler Blutdruckregelung ist in der (nicht vorveröffentlichten) Europäischen Patentanmeldung Nr. 06 112 431.9 beschrieben.

Die genannten Therapieeinrichtungen nutzen für die Blutdruckregelung grundsätzlich nur Werte und Zeitverläufe der vorangegangenen Therapievorgänge bis zum gegenwärtigen Behandlungszeitpunkt. Die in den Therapieeinrichtungen ebenfalls gespeicherten Werte und Zeitverläufe der vorangegangenen Therapievorgänge für die Zeitabschnitte nach dem gegenwärtigen Behandlungszeitpunkt bis zum Ende der Behandlung werden nicht in die Blutdruckregelung einbezogen.

Klinische Erfahrungen mit Dialysepatienten zeigen jedoch, dass sich aus diesen Zeitabschnitten vorangegangener Therapievorgänge wesentliche Informationen über den zu erwartenden Blutdruckverlauf nach dem jeweiligen Behandlungszeitpunkt der gegenwärtigen Behandlung ableiten lassen. Es erscheint aussichtsreich, dass sich daraus insbesondere für den selben Patienten bei vergleichbaren Behandlungsparametern, wie Gesamt-Ultrafiltrationsvolumen, maximale Ultrafiltrationsrate, Dialysatleitfähigkeit, Behandlungsdauer u.a., wichtige Schlussfolgerungen ergeben, die in der klinischen Praxis routinemäßig für eine vorausschauende Blutdruckregelung genutzt werden können. In Verbindung mit analytischen Vergleichen der Zeitverläufe des Blutdruckes sowie weiterer relevanter Einflussgrößen während der gegenwärtigen Behandlung und während vorangegangener Therapievorgänge anhand statistischer Ähnlichkeitskriterien ergeben sich günstige Voraussetzungen für die Voraussage bevorstehender Blutdruckabfälle.

Der Erfindung liegt deshalb die Aufgabe zu Grunde, eine Therapieeinrichtung derart auszubilden, dass durch die gedächtnisgestützte Regeleinrichtung eine Voraussage des bevorstehenden Blutdruckverlaufes und eine vorausschauende Blutdruckregelung ermöglicht werden.

Die Lösung dieser Aufgabe erfolgt erfindungsgemäß mit den Merkmalen des Patentanspruches 1. Hiernach führt die gedächtnisgestützte Regeleinrichtung nach jeder Blutdruckmessung die folgenden Funktionen aus:
a) Speicherung der Zeitverläufe des Blutdruckes sowie weiterer Einflussgrößen während der gegenwärtigen Behandlung (zum Beispiel Ultrafiltrationsrate, Ultrafiltrationsvolumen, Dialysatleitfähigkeit, relatives Blutvolumen);
b) Analytischer Vergleich der Zeitverläufe des Blutdruckes sowie weiterer relevanter Einflussgrößen während des gegenwärtigen Therapievorganges und während vorangegangener Therapievorgänge und Ermittlung vorangegangener Therapievorgänge mit hoher Ähnlichkeit anhand statistischer Ähnlichkeitskriterien;
c) Vorausschauende Blutdruckregelung zur Prävention von Blutdruckabfällen in einem nachfolgenden Zeitabschnitt.

Durch die erfindungsgemäße Einbeziehung der Zeitverläufe des Blutdruckes und weiterer relevanter Einflussgrößen aus vorangegangenen Therapievorgängen werden eine Voraussage bevorstehender Blutdruckabfälle und eine vorausschauende Blutdruckregelung auf der Grundlage patientenspezifischer Erfahrungen ermöglicht. Unter vergleichbaren Therapiebedingungen bezüglich der vom medizinischen Personal vorgegebenen Behandlungsmerkmale, wie Gesamt-Ultrafiltrationsvolumen, Therapiedauer, maximale Ultrafiltrationsrate, Dialysatleitfähigkeit und Grenzwerte des Blutdruck-Regelbereiches, wurden bei der Voraussage von bevorstehenden Blutdruckabfällen innerhalb des nachfolgenden Zeitabschnittes von z. B. 30 Minuten Trefferraten von über 85% erzielt.

Die Erfindung leistet insbesondere bei hypotonie-gefährdeten Patienten einen wesentlichen Beitrag zur Prävention von Blutdruckabfällen und damit zur Qualitätsverbesserung der Dialysebehandlung.

Vorzugsweise führt die gedächtnisgestützte Regeleinrichtung, die unter anderem auch die intervallartigen Blutdruckmessungen steuert, die folgenden Schritte aus:
d) Speicherung der Blutdruckmesswerte für eine festgelegte Anzahl von Therapievorgängen, einschließlich von zu festgelegten Zeitpunkten aus vorangegangenen Therapievorgängen in Form von Leitkurven übernommenen Substitutionswerten und der Führungsgrößen der gedächtnisgestützten Blutdruckregelung (zum Beispiel Ultrafiltrationsrate, Dialysatleitfähigkeit),
e) Speicherung weiterer Behandlungsparameter (zum Beispiel Gesamt-Ultrafiltrationsvolumen, maximale Ultrafiltrationsrate, Therapiedauer, Grenzwerte des Blutdruck-Regelbereiches) für die gemäß d) gespeicherten Therapievorgänge,
f) Analyse und Klassierung der unter d) genannten Blutdruckwerte für verschiedene Zeitabschnitte der vorangegangenen Therapievorgänge entsprechend ihrer Ähnlichkeit zum Blutdruckverlauf des gegenwärtigen Therapievorganges,
g) Ermittlung von Maßnahmen zur vorausschauenden Blutdruckregelung für die verwendeten Führungsgrößen anhand derjenigen vorangegangenen Therapievorgänge mit der größten Ähnlichkeit zum gegenwärtigen Blutdruckverlauf,
h) Anpassung der Regelcharakteristiken entsprechend den nach Schritt g) ermittelten Maßnahmen in Abhängigkeit vom gegenwärtigen Blutdruckverlauf und vom Zeitpunkt während des laufenden Therapievorganges.

Von der gedächtnisgestützten Regeleinrichtung werden die folgenden Kategorien von Blutdruckwerten in die vorausschauende Blutdruckregelung einbezogen:
i) Blutdruckwerte, die durch intervallartige Messungen gemäß dem in der Regeleinrichtung implementierten Zeitregime ermittelt werden, wie dies in EP 0 956 872 B1 beschrieben ist, und
j) Blutdruckwerte, die zwecks Reduzierung der Anzahl der Blutdruckmessungen in Form von Leitkurven aus vorangegangenen Therapievorgängen substituiert werden, wie dies in der Europäischen Patentanmeldung EP 1 226 838 A2 beschrieben ist, und
k) Blutdruckwerte, die von angeschlossenen Mess- und Auswertegeräten für andere physiologische Größen (zum Beispiel EKG, Relatives Blutvolumen, Hämatokrit, Blutsauerstoffgehalt) zu anderen Zeitpunkten als unter i) und j) automatisch angefordert werden, wie dies in der Europäischen Patentanmeldung 06 112 431.9 beschrieben ist, und
1) Blutdruckwerte, die vom medizinischen Personal manuell zu anderen Zeitpunkten als unter i) und j) angefordert werden, wie dies in der Europäischen Patentanmeldung 06 112 431.9 beschrieben ist.

Gemäß einer bevorzugten Weiterbildung der Erfindung ist vorgesehen, dass die gedächtnisgestützte Regeleinrichtung nach jeder Blutdruckmessung alle gespeicherten Therapieverläufe des jeweiligen Patienten hinsichtlich ihrer Ähnlichkeit zum gegenwärtigen Blutdruckverlauf analysiert. Um stets einen aktuellen Daten-Pool zu gewährleisten, ist die Speicherung von maximal 100 Behandlungen je Patient vorgesehen (n ≤ 100). Beim üblichen Behandlungsrhythmus von drei Dialysen je Woche repräsentiert der gespeicherte Daten-Pool die Behandlungen von bis zu 33 Wochen und somit maximal etwa acht Monate. Bei Speicherung der 101. Behandlung wird die 1. Behandlung automatisch gelöscht. Dadurch ist eine laufende Aktualisierung des Daten-Pools gewährleistet.

Gemäß einer bevorzugten Weiterbildung der Erfindung ist weiterhin vorgesehen, dass nach jeder Blutdruckmessung für die vorausschauende Blutdruckregelung eine bestimmte Anzahl von z. B. 3 bis 5 vorangegangenen Behandlungen mit der jeweils größten Ähnlichkeit zum gegenwärtigen Blutdruckverlauf analysiert werden. Dadurch wird gewährleistet, dass im Interesse einer möglichst hohen Voraussagewahrscheinlichkeit bevorstehender Blutdruckabfälle nur Behandlungen betrachtet werden, deren Ähnlichkeit sich markant von der Mehrzahl der gespeicherten Behandlungen abhebt. Die Ähnlichkeitsanalysen erfassen vorzugsweise die Zeitabschnitte vom Beginn der Behandlung bis zum gegenwärtigen Zeitpunkt oder nur 15 bis 20 Minuten vor dem gegenwärtigen Zeitpunkt oder eine Kombination beider Varianten.

Gemäß einer bevorzugten Weiterbildung der Erfindung ist weiterhin vorgesehen, dass die vorausschauende Blutdruckregelung aktiviert wird, sobald die Mehrzahl der analysierten 3 bis 5 vorangegangenen Therapievorgänge im nachfolgenden Zeitabschnitt von etwa 30 Minuten Blutdruckwerte innerhalb des aktiven Blutdruck-Regelbereiches aufweisen. Die gedächtnisgestützte Regeleinrichtung reagiert dann mit einer Reduzierung der Ultrafiltrationsrate und/oder Erhöhung der Dialysatleitfähigkeit.

Die Erfindung eignet sich insbesondere für Dialysegeräte und andere Geräte für die extrakorporale Blutreinigung, jedoch auch beispielsweise für die Infusionstherapie.

Im Folgenden wird unter Bezugnahme auf die Zeichnungen ein Ausführungsbeispiel der Erfindung näher erläutert.

Es zeigen:
- Fig. 1: eine schematische Darstellung eines Dialysegerätes,
- Fig. 2: ein Zeitdiagramm einer Dialysebehandlung (Auszug) ohne vorausschauende Blutdruckregelung, und
- Fig. 3: ein Zeitdiagramm einer Dialysebehandlung (Auszug) mit vorausschauender Blutdruckregelung.

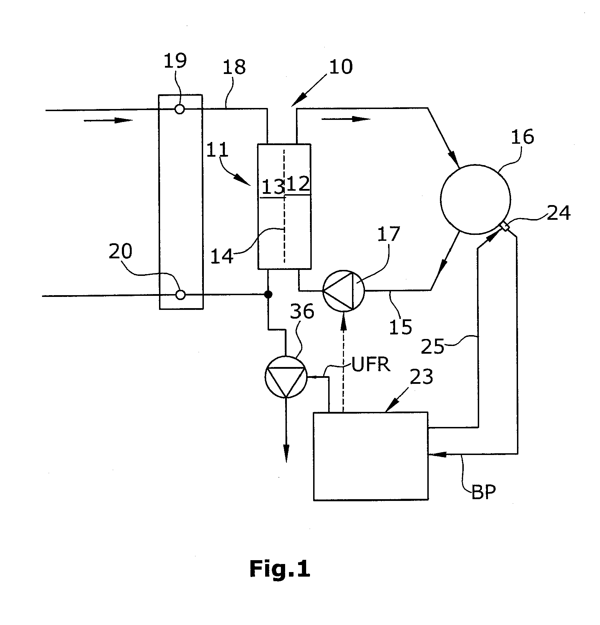

Das in Fig. 1 dargestellte Dialysegerät entspricht in seinem Grundaufbau dem von EP 0 956 872 B1 oder von EP 1 226 838 A2. Das Dialysegerät 10 enthält eine Ultrafiltrationsvorrichtung 11 mit einer Primärkammer 12 und einer Sekundärkammer 13, die durch eine Membran 14 voneinander getrennt sind. Die Primärkammer 12 ist Bestandteil eines Blutkreislaufes 15, bei dem Blut, welches über das arterielle System des Patienten entnommen wurde, in der Ultrafiltrationsvorrichtung 11 gereinigt und anschließend über das venöse System zum Patienten 16 zurückgeführt wird. In dem Blutkreislauf 15 befindet sich eine Pumpe 17. Diese ist als volumetrische Pumpe ausgebildet, d. h. die Fördermenge dieser Pumpe entspricht ihrer Antriebsgeschwindigkeit, und ist steuerbar.

Die Sekundärkammer 13 der Ultrafiltrationsvorrichtung 11 befindet sich in einem Dialysierflüssigkeitsweg 18, in dem Dialysierflüssigkeit gepumpt wird. Die Dialysierflüssigkeit kommt von einem (nicht dargestellten) Vorratsbehälter, nimmt in der Ultrafiltrationsvorrichtung 11 zusätzliche Substanzen aus dem Blut auf und wird dann zu einem (nicht dargestellten) Ablauf gepumpt. In dem Dialysierflüssigkeitsweg ist vor und hinter der Sekundärkammer 13 jeweils eine Durchflusskammer 19 bzw. 20 angeordnet, welche die Durchflussrate an der betreffenden Stelle regelt. Die Durchflusskammer 19 und die Durchflusskammer 20 haben die gleiche Förderrate. Über die volumengesteuerte Ultrafiltrationspumpe 36 wird die gewünschte Ultrafiltrationsmenge UFV in einer festgelegten Ultrafiltrationsrate UFR abgezogen. Das Zeitintegral über die Ultrafiltrationsrate UFR bildet das Ultrafiltrationsvolumen UFV, also dasjenige Flüssigkeitsvolumen, das seit Beginn der Behandlung die Membran 14 passiert hat. Die Regelung der Ultrafiltrationsrate erfolgt durch die Regeleinrichtung 23, die Steuersignale für die Förderrate der Pumpe 36 liefert. Die Förderrate der Pumpe wird in der Weise eingestellt, dass sich eine gewünschte Ultrafiltrationsrate ergibt.

Die Regeleinrichtung 23 empfängt ferner das Blutdrucksignal BP eines Blutdruckmessers 24, der am Körper des Patienten angebracht ist. Der Blutdruckmesser weist eine aufblasbare Manschette auf, die um den Arm des Patienten gelegt ist, und führt nicht-invasive Blutdruckmessungen aus. Die Zeitpunkte der Blutdruckmessungen werden intern durch die Regeleinrichtung 23 über die Leitungen 25 und extern durch angeschlossene Monitore und/oder das medizinische Personal festgelegt. Neben dem Blutdruckwert können der Regeleinrichtung 23 auch noch Blutdrucktrendwerte zugeführt werden wie es in EP 0 956 872 B1 beschrieben ist. Die Regeleinrichtung 23 regelt in Abhängigkeit von den erhaltenen Eingangsgrößen die Ultrafiltrationsrate UFR.

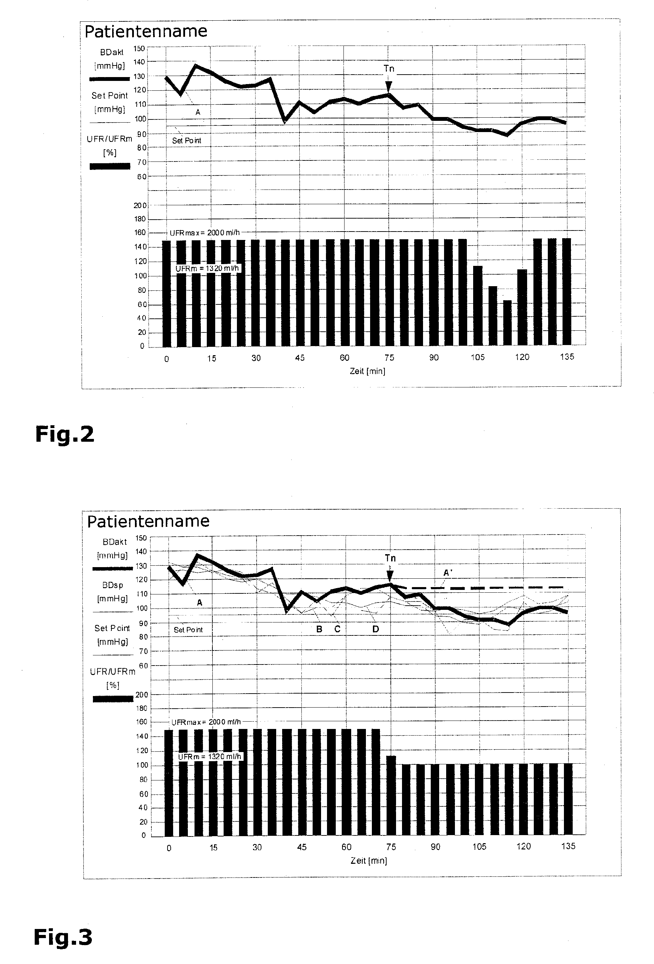

Fig. 2 veranschaulicht am Beispiel einer Dialysebehandlung die Blutdruckregelung, wie sie von der Regeleinrichtung 23 bisher ohne vorausschauende Blutdruckregelung gesteuert wird. Zu dem betrachteten Behandlungszeitpunkt Tn = 75 Minuten ergibt sich aus der hier durchgeführten Blutdruckmessung ein Blutdruck, der beträchtlich über dem oberen Grenzwert des aktiven Regelbereiches der Regeleinrichtung liegt. Dieser obere Grenzwert ist in Fig. 2 als Set Point eingezeichnet. Durch die Regeleinrichtung erfolgt deshalb anhand des Verlaufes der Blutdruckkurve A keine Reduzierung der Ultrafiltrationsrate, so dass die vom medizinischen Personal vorgewählte maximale Ultrafiltrationsrate UFRmax = 2000 ml/h aufrecht erhalten wird. UFRmax beträgt in diesem Beispiel etwa 150% der mittleren Ultrafiltrationsrate UFRm = 1320 ml/h, die sich aus dem vorgegebenen Dialyseziel (Gesamt-Ultrafiltrationsvolumen und Behandlungsdauer) ergibt. Der zu einem späteren Zeitpunkt bei 105 Minuten auftretende Blutdruckabfall unter den Set Point wird von der bisher verwendeten Regeleinrichtung ohne vorausschauende Blutdruckregelung nicht wahrgenommen, da von ihr gespeicherte Blutdruckverläufe vorangegangener Behandlungen nur bis zum jeweiligen Behandlungszeitpunkt (in Fig. 2: Tn = 75 Minuten) berücksichtigt werden. Die Regeleinrichtung beginnt deshalb in diesem Beispiel erst zum Zeitpunkt der Unterschreitung des Set Point, d. h. nach 105 Minuten, mit der Reduzierung der Ultrafiltrationsrate.

Fig. 3 veranschaulicht die Vorteile einer erfindungsgemäßen Regeleinrichtung mit vorausschauender Blutdruckregelung anhand des selben Blutdruckverlaufes A bis zum betrachteten Behandlungszeitpunkt Tn = 75 Minuten (min) wie in Fig. 2. Zusätzlich sind in Fig. 3 (dünn) die drei gespeicherten Blutdruckkurven B, C und D aus vorangegangenen Behandlungen eingezeichnet, die anhand der in der Regeleinrichtung implementierten Ähnlichkeitskriterien für den gegenwärtigen Behandlungsabschnitt von 0 bis 75 min die größte Ähnlichkeit zum aktuellen Blutdruckverlauf BDakt aufweisen. Die Ähnlichkeitskriterien ergeben sich hierbei aus statistischen Analysen der Blutdruckverläufe (beispielsweise Mittelwerte, Standardabweichungen, t-Tests, Mustererkennung).

Die gedächtnisgestützte Regeleinrichtung mit vorausschauender Blutdruckregelung erkennt jetzt bereits zum betrachteten Behandlungszeitpunkt Tn = 75 min, dass zwei der drei selektierten Blutdruckkurven aus vorangegangen Behandlungen im gewählten Voraussagezeitraum Tn+30min, d. h. im bevorstehenden Behandlungsabschnitt von 75 min bis 105 min, Blutdruckabfälle unter den Set Point aufweisen. Die Regeleinrichtung veranlasst deshalb jetzt bereits bei Tn = 75 min eine vorausschauende Reduzierung der Ultrafiltrationsrate mit dem Ziel, dadurch einen Blutdruckabfall unter den Set Point wie bei Kurve A in der gegenwärtigen Behandlung möglichst zu vermeiden oder zumindest zu minimieren. Diese Zielfunktion wird in Fig. 3 durch den hypothetischen (gestrichelten) Blutdruckverlauf A' angedeutet. Wesentliche Einflussgrößen der gegenwärtigen Behandlung und der selektierten Blutdruckverläufe B, C und D, wie beispielsweise Gesamtultrafiltrationsvolumen, Therapiedauer, maximale Ultrafiltrationsrate, oberer und unterer Grenzwert des Blutdruckregelbereiches, werden bei der Bemessung der jeweiligen Reduzierung der Ultrafiltrationsrate berücksichtigt.

Die gedächtnisgestützte Regeleinrichtung mit vorausschauender Blutdruckregelung wiederholt die in Fig.3 veranschaulichten Arbeitsschritte nach jeder Blutdruckmessung. Hierfür sind im implementierten Zeitregime in Abhängigkeit von der laufenden Behandlungszeit und dem bereits entzogenen Ultrafiltrationsvolumen Zeitabschnitte von 15min bis 30min festgelegt. Der tatsächliche Verlauf von A' während der laufenden Behandlung wird deshalb durch jede nachfolgende Blutdruckmessung im Sinne einer schrittweisen Optimierung der Behandlung modifiziert.

## Patentansprüche

1. Therapieeinrichtung für eine Behandlung mit einstellbaren Zielparametern wie Gesamt-Ultrafiltrationsvolumen, maximale Ultrafiltrationsrate, Behandlungsdauer, mit einem Blutdruckmessgerät (24) und einer gedächtnisgestützten Regeleinrichtung (23) zur Regelung des Blutdruckes des Patienten, wobei die gedächtnisgestützte Regeleinrichtung nach jeder Blutdruckmessung die folgenden Funktionen ausführt:
a) Speicherung der Zeitverläufe des Blutdrucks und weiterer Einflussgrößen während vorangegangener Behandlungen; Erfassung der Zeitverläufe des Blutdruckes sowie der weiteren Einflussgrößen während der gegenwärtigen Behandlung;
b) Analytischer Vergleich der Zeitverläufe des Blutdruckes und der weiteren Einflussgrößen während des gegenwärtigen Therapievorganges und während vorangegangener Therapievorgänge anhand von Ähnlichkeitskriterien und Ermittlung vorangegangener Therapievorgänge mit hoher Ähnlichkeit mit dem gegenwärtigen Therapievorgang;
c) Vorausschauende Blutdruckregelung zur Prävention von Blutdruckabfällen in einem nachfolgenden Zeitabschnitt.

2. Therapieeinrichtung nach Anspruch 1, wobei die gedächtnisgestützte Regeleinrichtung, die unter anderem auch die intervallartigen Blutdruckmessungen steuert, die folgenden Schritte ausführt:
- Speicherung der Blutdruckmesswerte für eine festgelegte Anzahl von Therapievorgängen einschließlich der zu festgelegten Zeitpunkten aus vorangegangenen Therapievorgängen in Form von Leitkurven übernommenen Substitutionswerte und der Führungsgrößen der Blutdruckregelung,
- Speicherung weiterer Behandlungsparameter für die gespeicherten Therapievorgänge,
- Analyse und Klassierung der gespeicherten Blutdruckwerte für verschiedene Zeitabschnitte der vorangegangenen Therapievorgänge entsprechend ihrer Ähnlichkeit zum Blutdruckverlauf des gegenwärtigen Therapievorganges,
- Ermittlung von Maßnahmen zur vorausschauenden Blutdruckregelung für die verwendeten Führungsgrößen anhand derjenigen vorangegangenen Therapievorgänge mit der größten Ähnlichkeit zum gegenwärtigen Blutdruckverlauf,
- Anpassung der Regelcharakteristiken entsprechend den genannten Maßnahmen in Abhängigkeit vom gegenwärtigen Blutdruckverlauf und vom Zeitpunkt während des laufenden Therapievorganges.

3. Therapieeinrichtung nach einem der Ansprüche 1 und 2, wobei für die Ermittlung der Ähnlichkeit vorangegangener Therapievorgänge mit dem gegenwärtigen Blutdruckverlauf die Zeitabschnitte vom Beginn des Therapievorganges bis zum gegenwärtigen Zeitpunkt und/oder kürzere Zeitabschnitte, zum Beispiel 15 Minuten, zugrunde gelegt werden.

4. Therapieeinrichtung nach einem der Ansprüche 1-3, wobei von der gedächtnisgestützten Regeleinrichtung alle oder einige der folgenden Kategorien von Blutdruckwerten in die vorausschauende Blutdruckregelung einbezogen werden:
- Blutdruckwerte, die durch intervallartige Messungen gemäß dem in der Regeleinrichtung implementierten Zeitregime ermittelt werden,
- Blutdruckwerte, die zwecks Reduzierung der Anzahl der Blutdruckmessungen in Form von Leitkurven aus vorangegangenen Therapievorgängen substituiert werden,
- Blutdruckwerte, die von angeschlossenen Mess- und Auswertegeräten für andere physiologische Größen (zum Beispiel EKG, Relatives Blutvolumen, Hämatokrit, Blutsauerstoffgehalt) automatisch angefordert werden, und
- Blutdruckwerte, die vom medizinischen Personal manuell angefordert werden.
